# EUROPEAN PATENT APPLICATION

(11) **EP 1 477 551 A1**
(43) Date of publication of application: **17.11.2004**
(21) Application number: 03731728.6
(22) Date of filing: 24.01.2003
(51) Int. Cl.: C11C 3/04, C11C 3/10, C07C 69/24, C07C 67/03

(54) **METHOD FOR THE TRANS-ESTERIFICATION OF TRIGLYCERIDES WITH MONOALCOHOLS HAVING A LOW MOLECULAR WEIGHT IN ORDER TO OBTAIN LIGHT ALCOHOL ESTERS USING MIXED CATALYSTS**

(30) Priority: 25.01.2002 ES 200200173
(71) Applicant: UNIVERSIDAD COMPLUTENSE DE MADRID, E-28040 Madrid (ES); Instituto para la Diversificacion y Ahorro de la Energia, 28046 Madrid (ES)
(72) Inventor: ARACIL MIRA, J., 28040 Madrid (ES); MARTINEZ RODRIGUEZ, 28040 Madrid (ES); BAUTISTA SANTA CRUZ, 28040 Madrid (ES); GUIJARRO GIL, Maria Isabel, 28040 Madrid (ES)
(74) Representative: Carpintero Lopez, Francisco
(86) International application number: PCT/ES2003/000041
(87) International publication number: WO 2003/062358

(57) **Abstract**

The transesterification of triglycerides obtained from vegetable or animal oils or with used frying oils to obtain fatty acid esters of low molecular weight alcohols has been carried out with a mixed catalytic system made up of carboxylic acid salts and alkaline metal hydroxides. With this catalytic system the preliminary preparatory steps can be eliminated and the operation time reduced.

The process is carried out at moderate conditions of pressure and temperature, in a stirred-tank reactor, in continuous or discontinuous mode.

The esters obtained can be used as fuels and solvents in addition to other uses.

## Description

### INTRODUCTION

The present invention refers to a process to transesterify triglycerides of animal or plant origin or used frying oils to obtain esters of fatty acids of low molecular weight alcohols. Salts of carboxylic acids and alkaline metal hydroxides have been used as a catalytic system.

The importance of this invention lies in the use of the excess acidity, presented by all oils and fats of plant and animal origin, to obtain the catalytic system (carboxylic acid salts), eliminating preliminary preparatory steps of the raw materials and reducing the costs of the catalyst and the time previously required for the purification steps.

The process is carried out in moderate pressure and temperature conditions. In this process, the reaction is carried out in continuous and discontinuous modes in a stirred-tank reactor. The complete ester production process can be carried out in a discontinuous or continuous mode.

In this process, the reaction is carried out with a catalytic system, partly formed by the carboxylic acids found in free form in the raw material, permitting the preliminary preparatory steps to be diminished and also the operation time of the process.

### STATE OF THE ART OF THE TECHNIQUE

For decades, fatty acid esters have been the most important compounds in the fat industry and in oil chemistry, because of their important role as intermediate products in the production of other esters, amines, fatty alcohols and soaps, among others. Excellent applications have been found for these fatty acid esters in the food, textile and cosmetic industry and in the manufacture of synthetic lubricants.

Another aspect that can increase the interest for this group of products is the use of methylic and ethylic fatty acid esters as a clean alternative to diesel from the petrochemical industry. This type of ester is known as Biodiesel (DUNNE, W. "The production of fuels from biological substances". Int. I. Energy Res., Vol. 18, p. 71-78, 1994.; PRYDE, E.H. "Vegetable oils as fuel alternatives". JAOCS, Vol. 61, p. 1609-1610, 1984). Biodiesel, obtained in this way, presents excellent environmental benefits, especially in relation to the emission of SO₂, particles and smoke.

Low molecular weight alkyl esters of fatty acids can be prepared by direct reaction between triglycerides and the corresponding alcohol. The use of rapeseed and sunflower oils as sources for these reactions is especially interesting because they use production surpluses which, according to European legislation, cannot be used as food products. The transesterification reaction can be carried out by homogeneous classical catalysis, which is usually basic and heterogeneous or can use biocatalysts.

The catalytic transesterification of plant oils has been described since the middle of the XIX Century. In 1852, Duffy (DUFFY, P. "On the constitution of stearine". J. Chem. Soc., Vol. 5, p. 303, 1852) presented a procedure that used sodium methoxide as a catalyst. However, more studies were published on this area of research around the mid XX century. The use of different raw materials has also been studied in numerous research projects.

In 1984, Freedman et al. (FREEDMAN, B.; PRYDE, E.H. and MOUNTS, T.L. "Variables affecting the yields of fatty esters from transesterified vegetable oils". JAOCS, Vol. 61, p. 1638-1643, 1984), studied the alcoholysis of oils from cotton seed, peanut, soya and sunflower with monoalkylic alcohols using basic catalysts, sodium hydroxide and methoxide, at 60 °C. The frying oil used as raw material in Austria to obtain methylic esters with basic catalysts (FRÖLICH, A. and RICE, B. "The preparation and properties of biodiesel grade methyl ester from waste cooking oil". Minutes of the activity meeting of the IEA, Vienna, p. 11-18, 1995) and more recently alcoholysis of animal tallow has been described (MA, F.; CLEMENTS, L.D. and HANNA, M.A. "Biodiesel fuel from animal fat. Ancillary studies on transesterification of beef tallow". Ind. Eng. Chem. Res., Vol. 37, p. 3768-3771, 1998).

There is also information published in the literature about the different catalytic systems used in transesterification processes. In 1995, a process patented by Wimmer Theodor et al. U.S. Patent n° 5434279 was described, which used basic alkaline compounds or compounds of transition metals such as oxides, hydroxides, alcoholates, borates, carbonates and silicates of lithium, sodium, potassium and calcium as catalysts.

In the process patented by Chien Hoang et al. in the French Patent FR2698101, other heterogeneous catalytic systems and a variety of mixed catalysts were used. Hence, starting with potassium carbonate-mixtures of sodium carbonate-potassium bicarbonate-sodium-potassium carbonate and/or sodium and potassium phosphates among others, were made up.

As well as those described above, other catalytic systems were used in transesterification processes. The patent from 1985, Volpenheim. U.S. Patent n° US4517360, describes the use of alkaline metal salts of polyols in high concentrations in transesterification reactions, without solvent, of complex alkyl esters using basic catalysts such as metals, alloys and alkoxides among others. Among the polyols, monosaccharaides, disaccharides and other sugar alcohols are important.

The kinetic study of the esterification reaction of glycerine with fatty acids in the presence of sodium and potassium soaps was published in 1998 by Szelag et al. (SZELAG, H. and ZWIERZYKOWSKI, W. "Esterification kinetics of glycerol with fatty acids in the presence of sodium and potassium soaps". Fett-Lipid, Vol. 100 (7), 302-307, 1998). This shows that the rate constant of the reaction increases with increasing concentration of the soap in the reaction medium.

The Cognis process, EP1092703 for the production of methylic fatty acids from triglycerides is comprised of two steps. The first takes place at high pressures and temperatures in the presence of salts of transition metals as catalyst, such as magnesium stearate. The second step is produced after elimination of glycerine and consists in transesterification at low pressure in the presence of sodium methoxide.

### DESCRIPTION OF THE INVENTION

Procedure to transesterify triglycerides with low molecular weight monoalcohols to obtain light alcohol esters using mixed catalysts.

The present invention describes a new semi-continuous manufacturing procedure for light alcohol esters from fatty acids of raw, refined or used plant oils and other triglyceride mixtures by a transesterification process. In this process, a mixed system comprised of sodium or potassium soap obtained from the fatty acid of raw, refined or used vegetable oils of plant or animal fat or other triglyceride mixtures and those corresponding to sodium and potassium hydroxides are used as the catalytic system. These esters are used as fuel in diesel engines and also as fuel for domestic use.

The present invention refers to the use of mixed catalytic systems formed of soap and the corresponding sodium and potassium hydroxide, to direct the transesterification of light esters of fatty acids of raw, refined or used vegetable oils, of plant oils or animal fats and other triglyceride mixtures to obtain an ester yield above 99%, using a semicontinuous process in which the time required to obtain this yield is from 5 to 300 minutes although, normally, this time is between 60 and 150 minutes.

The transesterification reaction takes place according to conventional processes in a continuous or discontinuous reactor of the stirred―tank type, to which the catalytic system is added.

The reaction is carried out in a temperature interval of 10°C to 100°C, preferably between 15°C and 65°C, and at atmospheric pressure. The transesterification reaction can be carried out in one or two steps.

If the reaction is carried out in one step, the amount of alcohol used for the transesterification varies within an interval from 70% to 250% of the stechiometric value, preferably between 100% and 200%.

If the reaction is carried out in two steps, in the first step an amount of alcohol between 70% and 200% of the stechiometric amount, preferably between 70% and 175% is added to the reactor. Then, the reaction mixture is decanted to eliminate the glycerine formed, which is transferred to the glycerine tank, and the amount of alcohol required for this to be present in a stechiometric quantity between 100 % and 250%, preferably between 100% and 200%, is added to the reactor. Then, the reaction mixture is transferred to a separation system if operating in a continuous reactor or a deposit chamber for its purification if operating in a discontinuous reactor.

In the purification steps after the transesterification reaction, the mode of operation is continuous, independently of how the reaction system has been operated.

The catalytic system used for the transesterification reaction is a mixed system comprised of alkaline metal hydroxides, preferably of sodium and/or potassium, and of alkaline metal soaps, preferably of sodium and/or potassium, obtained from free fatty acids contained in the raw materials via a process of neutralization. The neutralization of these fatty acids is done using non aqueous solutions of alkaline metal hydroxides, preferably of sodium and/or potassium, producing the corresponding soaps that act as promoters or coadjuvants of the transesterification reaction.

Depending on the acidity of the starting material and the amount of soaps required for the transesterification reaction, it will be necessary to add an amount of alkaline metal hydroxides between 0.1 and 1.0% in weight of the reaction mass, preferably between 0.2 and 0.8%.

The reaction system formed by stirred-tank reactors is equipped with a filtered system in order to maintain in the reaction medium the largest amount of soaps, since these favor the progress of the transesterification reaction.

If the transesterification process is carried out in a discontinuous process, the excess methanol or ethanol will be separated from the reaction medium by vacuum distillation. If the transesterification reaction is conducted in continuous mode, the excess methanol and ethanol will be separated from the reaction medium by vacuum distillation from the deposit chamber which feeds the separation and purification processes of the biodiesel. In both cases, the methanol or ethanol recovered will be transferred to the corresponding methanol or ethanol storage tank.

The transesterification mixture, both if the reaction is carried out in discontinuous mode or in continuous mode, will be transferred to a deposit chamber which, if the reaction is conducted in continuous mode, will be equipped with a vacuum distillation system to eliminate the excess methanol or ethanol from the reaction. This deposit chamber, which will have between 2 and 15 times the volume of the transesterification reactor, usually between 4 and 7 times, will continually feed the biodiesel and glycerine separation and purification system.

The mixture from the deposit chamber will continually feed a separation system in which two differentiated phases are obtained: the ester phase (A) and the glycerinous phase (B).

To the ester phase (A), mainly comprised of the light alcohol ester used, the catalyst and traces of unreacted alcohol, glycerine and soaps, will be rinsed with water and/or a diluted solution of a strong acid, such as H₂SO₄, H₃PO₄, HCl, etc., and, then, the two phases formed will be separated. One of these phases (A1), will mainly be comprised by the ester of the alcohol used for the transesterification. The other aqueous phase (A2), will be submitted to a neutralization operation so that it complies with environmental rules and regulations. These rinsing waters will be vacuum distilled to recover any remains of methanol they may contain and transferred to the corresponding tank of raw material.

Then, in the (A1) phase the organic compound is added so that the biodiesel formed complies with specifications of the parameter "cold filter obturation point" (CFOP). After this, it is dried to eliminate any traces of water or alcohol that may remain.

Next, this current with the (A1) phase, free of moisture and alcohol, is filtered to eliminate any solid particles it may contain, either from the raw material or from the catalytic system used.

The product obtained after the filtration step is an alkyl ester that fulfils specifications of the European Union for its use as biodiesel.

The glycerinous phase (B) is submitted to vacuum distillation to eliminate the remains of methanol or ethanol and is then submitted to neutralization with H₂SO₄, H₃PO₄, HCl, etc., preferably with H₃PO₄. Next, the three phases formed are separated: the liquid phase (B1) of glycerine with a purity between 86% and 92%, the liquid phase (B2) of fatty acids (B2) and the solid phase (B3) of the sodium or potassium salt of the acid used in neutralization of the glycerinous phase.

### APPLICATION OF THE INVENTION

The present invention is also illustrated by the following examples:

### EXAMPLE 1

This example shows the influence of the activity of the catalytic system using potassium hydroxide as the only component of this catalytic system.

The reaction was carried out in a 125 ml volume complete mixture reactor equipped with a stirrer at 600 rpm where the reaction temperature is maintained constant at 25°C and the working pressure at atmospheric pressure.

A total of 53.40 g of the triglyceride raw material with an acidity of 10%, 14.60 g methanol and 0.2265 g potassium hydroxide (0.40% in weight, 4.04 milliequivalents) were added to the reactor. After 60 minutes of reaction, conversion of the triglyceride raw material was 99% and the yield in methylic esters was 95% (Yield = kg of methyl ester produced/100 kg of triglyceride raw material added).

### EXAMPLE 2

This example shows the influence of the activity of the catalytic system obtained by neutralization with potassium hydroxide of the free fatty acids present in the starting material that corresponds to the only component of the catalytic system.

The reaction is carried out in a 125 ml volume complete mixture reactor with a 600 rpm stirrer system where the reaction temperature is maintained constant at 25°C and the working pressure at atmospheric pressure.

A total of 53.40 g of the triglyceride starting material with an acidity of 10% is added to the reactor and 14.60 g of methanol. Neutralization is done stechiometrically with 8.10 milliequivalents of potassium hydroxide, obtaining 2.6 g potassium soaps, and the transesterification reaction begins. After 60 minutes reaction, conversion of the triglyceride starting material was 18% and the yield of methylic esters was 15%.

### EXAMPLE 3

This example shows the effect of the potassium hydroxide milliequivalents on the composition of the catalytic system.

The following table shows the experiments carried out in the same working conditions (type of reactor, temperature, pressure, agitation, amount of reactants and operation time) as examples 1 and 2, changing the acidity of the triglyceride raw material to be transesterified (between 0% and 10%).

**Table I**

| Assay | Acidity starting material | Potassium soap | | KOH | | | Total | Convesion | Yield |
|---|---|---|---|---|---|---|---|---|---|
| (n°) | (%) | (g) | (meq) | (g) | (meq) | (%) | (meq) | (%) | (%) |
| 3 | 10.0 | 2.6 | 8.10 | 0.226 | 4.04 | 0.40 | 12.14 | 99 | 95 |
| 4 | 5.0 | 1.3 | 4.05 | 0.226 | 4.04 | 0.40 | 8.09 | 97 | 93 |
| 5 | 1.0 | 0.26 | 0.80 | 0.460 | 8.10 | 0.81 | 9.00 | 100 | 96 |
| 6 | 1.2 | 0.32 | 0.99 | 0.138 | 2.46 | 0.24 | 3.45 | 75 | 72 |
| 7 | 0.7 | 0.23 | 0.71 | 0.223 | 3.97 | 0.39 | 4.68 | 96 | 92 |
| 8 | 0.4 | 0.14 | 0.43 | 0.314 | 5.60 | 0.56 | 6.03 | 100 | 96 |
| 9 | 1.2 | 0.32 | 0.99 | 0.0 | 0.00 | 0.00 | 0.99 | 7 | 4 |
| 10 | 0.0 | 0.0 | 0.00 | 0.138 | 2.46 | 0.24 | 2.46 | 68 | 65 |
| 11 | 0.4 | 0.14 | 0.43 | 0.0 | 0.00 | 0.00 | 0.43 | 4 | 3 |
| 12 | 0.0 | 0.0 | 0.00 | 0.223 | 3.97 | 0.39 | 3.97 | 95 | 91 |
| 13 | 10.0 | 2.6 | 8.10 | 0.0 | 0.00 | 0.00 | 8.10 | 18 | 15 |

As can be observed in the table, use of the mixed catalytic system gives a higher yield of methylic esters in the reaction, compared to that obtained with potassium hydroxide as the only component of the catalytic system, at the same milliequivalents of potassium hydroxide used.

### EXAMPLE 4

This example shows the influence of the reaction temperature.

Assay number 3, shown in Table I, was repeated with the same catalytic system and working conditions except for the reaction temperature, which was maintained constant at 65°C. After 60 minutes of reaction, conversion of the triglyceride starting material was 100%, with a yield of methylic esters of 91%.

### EXAMPLE 5

This example shows the influence of reaction time.

Assay number 3, shown in Table I, was repeated with the same catalytic system and working conditions with the exception of reaction time, which was 20 minutes. Conversion of the triglyceride starting material was 90%, with a yield of methylic esters of 86%.

### EXAMPLE 6

This example shows the influence of reaction time.

Assay number 3, shown in Table I, was repeated with the same catalytic system and working conditions except for reaction time. After 120 minutes of reaction, conversion of the triglyceride starting material was 100%, with a yield of methylic esters of 96%.

### EXAMPLE 7

This example shows the influence of stirring rate.

Assay number 3, shown in Table I, was repeated with the same catalytic system and working conditions except for stirring rate, which was 100 rpm. After 60 minutes, conversion of the triglyceride starting material was 40%, with a yield of methylic esters of 35%.

### EXAMPLE 8

This example shows the influence of the type of cation present in the hydroxide that will form part of the catalytic system.

Assay number 3, shown in Table I, was repeated with the same catalytic system and working conditions except for potassium hydroxide, which was replaced by sodium hydroxide. After 60 minutes, conversion of the triglyceride starting material was 99%, with a yield of methylic esters of 93%.

### EXAMPLE 9

This example shows the mode of operation of the transesterification process.

In a 125 ml volume complete mixture tank reactor, equipped with a stirring system, 53.40 g of triglyceride starting material, with an acidity of 0.4%, and 7.25 g of methanol were introduced. The reaction temperature was maintained constant at 25°C, at atmospheric pressure and a stirring rate of 600 rpm. Neutralization was done stechiometrically with 0.71 milliequivalents of potassium hydroxide. Then, 0.32 g potassium hydroxide were added to the reaction mixture to complete the mixed catalytic system.

After 20 minutes, the glycerine formed in the transesterification is separated, obtaining 80% conversion of the triglyceride starting material. Next, 2.90 g methanol are added to the reaction system and after 20 minutes 99% conversion of the triglyceride starting material was obtained, with a yield of methylic esters of 96%.

### EXAMPLE 10

This example shows the influence of the molecular weight of the alcohol used in the transesterification reaction.

Assay number 3, shown in Table I, is repeated with the same catalytic system and working conditions except that methylic alcohol was replaced by ethylic alcohol in the transesterification process. After 60 minutes, conversion of the triglyceride starting material was 38%, with a yield of methylic esters of 35%.

### EXAMPLE 11

This example shows the mode of operation.

The reaction is carried out in a 2000 ml complete mix reactor equipped with an 800 rpm stirring system where the reaction temperature is kept constant at 25°C and the working pressure at atmospheric pressure.

A total of 842.6 g of the triglyceride starting material for transesterification with an acidity of 3% is added to the reactor. Next, a solution containing 5.0 g potassium hydroxide in 88.7 g methanol is added. At this moment, the free fatty acids are neutralized forming the corresponding potassium soap. After this step, 5.3 g of dissolved potassium hydroxide are added dissolved in 88.7 g methanol. The mixture is stirred, triggering the transesterification reaction. After 60 minutes., the reaction is completed and the two liquid phases that have formed are separated.

The ester phase (A), contains 810.5 g of fatty acid methylic ester, 45.1 g methanol, 3.8 g triglycerides, 2.9 g fatty acids and 0.3 g potassium soap. The glycerinous phase (B), contains 85.0 g glycerine, 44.8 g methanol, 39.7 g potassium soap, 5.2 g potassium hydroxide and 0.2 g methylic esters.

The ester phase is rinsed using 409.1 g water. The two immiscible phases formed are separated, obtaining the following composition for each of them. The ester phase (A1) contains 808.7 g methylic esters, 3.8g triglycerides and 2.9 g fatty acids. The aqueous phase (A2) contains 409.1 g water, 45.1 g methanol, 1.6 g methyl esters and 0.3 g potassium soaps. The aqueous phase (A2) is then submitted to distillation at 25°C and a pressure of 16 mmHg, collecting the 45.1 g methanol it contained.

The glycerinous phase (B), is also submitted to distillation at 25°C and a pressure of 16 mmHg, collecting the 44.8 g methanol it contained. Next, a solution of 21.4 g ortho-phosphoric acid in 9.1 g water is added to the glycerinous phase (B) devoid of methanol. In this process, three different phases are formed: two liquid phases and one precipitated solid phase. These three phases are separated obtaining the following composition for each of them. The (B1) phase contains 85.0 g glycerine, 9.1 g water and 0.2 g methylic esters. Phase (B2) is composed of 34.9 g fatty acids and phase (B3) is composed of 27.6 g of a precipitate comprised of potassium phosphate salts.

## Claims

1. Process to transesterify triglycerides with low molecular weight monoalcohols to obtain light alcohol esters using mixed catalysts **characterized in that** the triglycerides come from raw, refined or used vegetable oils, plant oils or animal fats, and mixtures thereof.

2. Process to transesterify triglycerides with low molecular weight monoalcohols to obtain light alcohol esters using mixed catalysts, according to the previous claim, **characterized in that** the monoalcohols are preferably methanol and ethanol.

3. Process to transesterify triglycerides with low molecular weight monoalcohols to obtain light alcohol esters using mixed catalysts, according to the previous claim, **characterized in that** the mixed catalytic system is comprised of alkaline metal hydroxides, preferably sodium and potassium, and of soaps obtained from free fatty acids contained in the starting materials by a process of neutralization.

4. Process to transesterify triglycerides with low molecular weight monoalcohols to obtain light alcohol esters using mixed catalysts, according to claim 3, **characterized in that** the neutralization of the acidity of the raw materials is performed by the alkaline hydroxides forming the corresponding salts of carboxylic acids employed in the catalytic system.

5. Process to transesterify triglycerides with low molecular weight monoalcohols to obtain light alcohol esters using mixed catalysts, according to claims 3 and 4, **characterized in that** the amount of alkaline hydroxides used, ranges between 0.1 and 1% of the reaction mass.

6. Process to transesterify triglycerides with low molecular weight monoalcohols to obtain light alcohol esters using mixed catalysts, according to previous claims, **characterized in that** it uses continuous or discontinuous reactors, of the stirred-tank type, equipped with a filtration system at the exit of the reaction system.

7. Process to transesterify triglycerides with low molecular weight monoalcohols to obtain light alcohol esters using mixed catalysts, according to previous claims, **characterized in that** the transesterification reaction is carried out at a temperature between 10° and 100°C and in a time between 5 and 300 minutes.

8. Process to transesterify triglycerides with low molecular weight monoalcohols to obtain light alcohol esters using mixed catalysts, according to previous claims, **characterized in that** the transesterification reaction is carried out in one step introducing in the reactor an amount of alcohol ranging from 70 to 250% of the stechiometric amount.

9. Process to transesterify triglycerides with low molecular weight monoalcohols to obtain light alcohol esters using mixed catalysts, according to claims 1 to 7, **characterized in that** the transesterification reaction is carried out in two steps:
1. An amount of alcohol between 70 and 200% of the stechiometric amount is introduced in the reactor.
2. The glycerine formed is separated from the reaction mixture and the required amount of alcohol is added to the reactor for this to encounter an amount between 100 and 250% of the stechiometric value.

10. Process to transesterify triglycerides with low molecular weight monoalcohols to obtain light alcohol esters using mixed catalysts, according to previous claims, **characterized in that** the transesterification reaction is carried out in a discontinuous process in which the excess methanol or ethanol is separated from the reaction medium by a vacuum distillation operation.

11. Process to transesterify triglycerides with low molecular weight monoalcohols to obtain light alcohol esters using mixed catalysts, according to claims 1 to 9, **characterized in that** the transesterification reaction is carried out in a continuous process in which the excess methanol or ethanol is separated from the reaction medium by vacuum distillation from the deposit chamber.

12. Process to transesterify triglycerides with low molecular weight monoalcohols to obtain light alcohol esters using mixed catalysts, according to previous claims, **characterized in that** the reaction mixture is transferred to a deposit chamber to be subsequently purified and obtain the light alcohol esters (biodiesel) and by-products, which are made after this time in continuous mode.

13. Process to transesterify triglycerides with low molecular weight monoalcohols to obtain light alcohol esters using mixed catalysts, according to the previous claim, **characterized in that** the mixture from the deposit chamber continuously feeds a separation system from which two phases are obtained: (A) the ester phase, mainly comprised of the ester, alcohols and soaps, and (B) the glycerine phase mainly comprised of glycerine, alcohol and soaps.

14. Process to transesterify triglycerides with low molecular weight monoalcohols to obtain light alcohol esters using mixed catalysts, according to the previous claim, **characterized in that** the ester phase is rinsed with water or with a diluted solution of strong acid, being separated into:
(A1). A phase comprised of the alcohol ester used for transesterification, to which an organic compound is added, to proceed to a drying operation followed by a filtration process obtaining an alkyl ester suitable for use as a combustion agent, fuel or solvent.
(A2). An aqueous phase submitted to a neutralization operation followed by vacuum distillation.

15. Process to transesterify triglycerides with low molecular weight monoalcohols to obtain light alcohol esters using mixed catalysts, according to claim 13, **characterized in that** the glycerine phase (B) is submitted to a vacuum distillation operation followed by neutralization with strong acid, preferably *ortho*-phosphoric acid giving rise to three by-products:
(B1). Liquid phase that contains glycerine
(B2). Liquid phase that contains fatty acids
(B3). Solid phase that contains a sodium or potassium salt of the acid used in the neutralization.

16. Use of the esters obtained with the claimed transesterification process as combustion agents, fuels and solvents.
